# EUROPEAN PATENT APPLICATION

(11) **EP 1 813 226 A1**
(43) Date of publication of application: **01.08.2007**
(21) Application number: 07100962.5
(22) Date of filing: 23.01.2007
(51) Int. Cl.: A61F 2/08

(54) **Anchor for tendons used in the reconstruction of a ligament, particularly of the cruciate ligamnet of the knee**

(30) Priority: 27.01.2006 IT BO20060008 U
(71) Applicant: CITIEFFE S.r.l., 40012 Calderara di Reno (Bologna) (IT)
(72) Inventor: Mingozzi, Franco, 40012, Calderara di Reno BO (IT); Dovesi, Alan, 40138, Bologna (IT); Aglietti, Paolo, 50123, Firenze (IT)
(74) Representative: Modiano, Micaela Nadia

(57) **Abstract**

An anchor for tendons used in the reconstruction of a ligament, particularly of the cruciate ligament of the knee, comprising a ring element (2) which can be inserted in a bore formed in the tibia or in the femoral condyle of the knee and an annular flange (3) for abutment against the region that surrounds the opening of the bore in the condyle in which the ring element (2) has been inserted, the ring element (2) being provided, on opposite sides, with two portions (8, 9) which protrude from it and are mutually aligned so as to be external to the cortex when the ring element is inserted in the bore, the annular flange (3) being provided with an opening (11) whose breadth is such as to allow the ring element (2) to pass through it and allow the protrusions (8, 9) to engage on the flange (3) in diametrically opposite positions.

## Description

The present invention relates to an anchor for tendons used in the reconstruction of a ligament, particularly of the cruciate ligament of the knee.

As is known, the cruciate ligament of the knee comprises two bundles: the anteromedial bundle and the posterolateral bundle.

The technique for reconstruction of the anterior cruciate ligament of the knee, known for example from EP-860,146, entails providing a bore which passes through the tibia, in alignment with a bore which passes through the femoral condyle. Two tendons, known as gracilis and semitendinosus, are inserted through the inlet of the tibial bore. Once these two tendons have exited from the opening of the femoral bore, they are passed through an anchor and reinserted first through the femoral bore and then through the tibial bore so that they can be pulled and anchored to the tibial cortex for example by means of one or more staples.

In order to allow adequate abutment of the anchor on the femoral cortex, the anchor is constituted by a cylindrical body, which is inserted in the femoral bore and is provided with an eye for the passage of the two tendons and with a flange for retention on the femoral cortex.

The known anchor has the substantial drawback that since the surface of the cortex is inclined with respect to the axis of the bore in which the cylindrical body of the anchor is to be inserted, the flange cannot rest fully on the surface of the cortex. Indeed, by resting against the cortex only with a small angular portion of the perimetric rim, it assumes an inclined and unstable arrangement which can compromise the subsequent step of tendon tensioning.

The aim of the present invention is to provide an anchor which allows to obviate the above-mentioned drawbacks.

Within this aim, an object of the present invention is to provide an anchor which is capable of facilitating the operating technique for its installation.

This aim and this and other objects which will become better apparent hereinafter are achieved by an anchor for tendons used in the reconstruction of a ligament, particularly of the cruciate ligament of the knee, characterized in that it comprises a ring element which can be inserted in a bore formed in the tibia or in the femoral condyle and an annular flange for abutment against the region that surrounds the opening of the bore in which said ring element has been inserted, said ring element being provided, on opposite sides, with two portions which protrude from it and are mutually aligned so as to be external to said bore when said ring element is inserted in said bore, said annular flange being provided with an opening whose breadth is such as to allow said ring element to pass through it and allow said protrusions to engage on said flange in diametrically opposite positions.

Further characteristics and advantages of the anchor according to the invention will become better apparent from the following detailed description of an embodiment thereof, illustrated only by way of nonlimiting example in the accompanying drawings, wherein:
Figure 1 is a perspective view of an anchor according to the invention;
Figure 2 is a side view of the anchor of Figure 1;
Figure 3 is a side view, rotated through 90° with respect to Figure 2;
Figure 4 is a plan view of the anchor of Figure 1;
Figure 5 is a perspective view of the ring element of the anchor;
Figure 6 is a side view of the ring element;
Figure 7 is a perspective view of the flange;
Figure 8 is a plan view of the flange;
Figure 9 is a view of the anchor installed for reconstruction of the cruciate ligament of the knee.

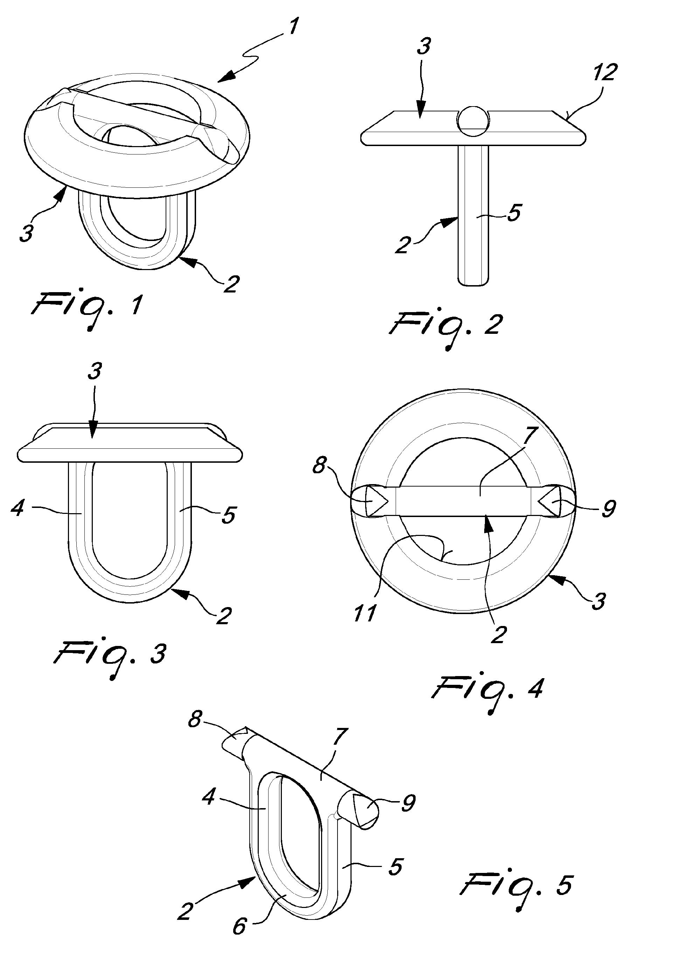

With reference to Figures 1 to 6, an anchor is generally designated by the reference numeral 1 and comprises a ring element 2 and an annular flange 2 which can be mutually associated in the manner specified below.

The ring element 2 forms an eye which has substantially the shape of an ellipse which is elongated in the direction A and is composed of two portions 4 and 5 which are straight and parallel and are mutually connected by two semicircular portions 6, 7.

Two portions 8, 9 extend in mutually opposite directions from the semicircular portion 7, are mutually aligned and beveled at their ends, and lie on the plane of the eye 2. The portions 8, 9 are shaped like cylindrical rods which are tangent to the semicircular portion 7 and are perpendicular to the straight portions 4, 5 and have a circular cross-section whose diameter is equal to the thickness of the portions 4-7. Conveniently, the cross-sections of the portions 4-7 are rounded elliptically in order to avoid the presence of edges or the like which might damage the tendons which will be passed through the eye 2 during ligament reconstruction.

The annular flange 3 is constituted by a washer 10, which encloses a central opening 11 whose diameter is slightly larger than the external distance between the parallel portions 4, 5 of the element 2. The washer 10 has a flat lower face for resting against the cortex and a cambered upper face 12 in which there are two notches 13, 14 which are mutually aligned diametrically.

The notches 13, 14 are substantially semicylindrical and have such dimensions as to act as seats adapted to accommodate articulately the rod-like portions 8, 9 of the ring element 2 when the element 2 and the flange 3 are mutually assembled to be installed in the bore provided in the corresponding femoral or tibial condyle.

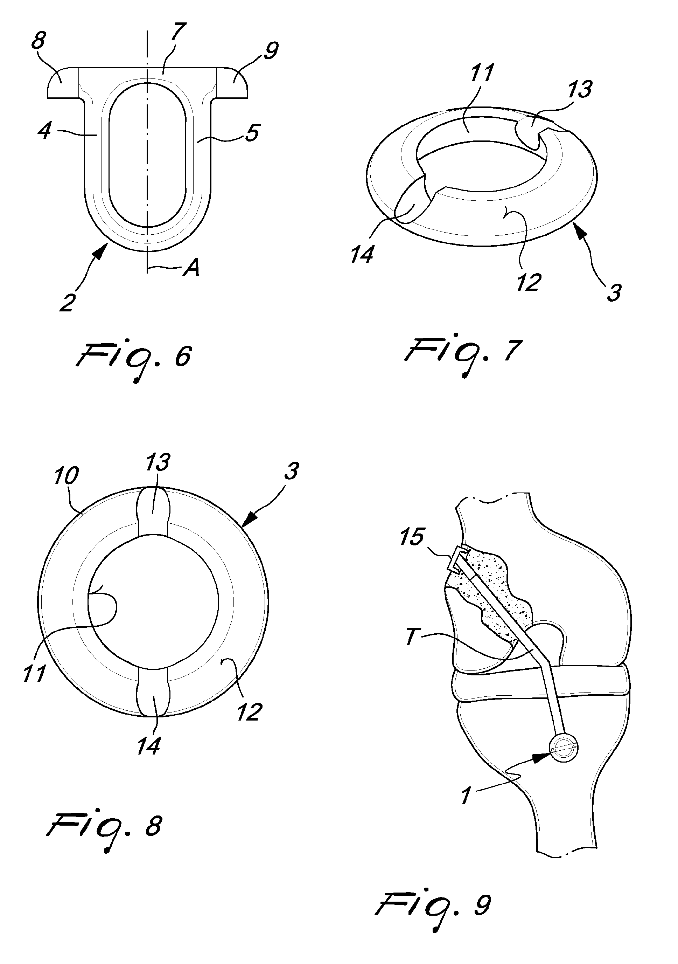

The method for installing the described anchor 1 can be deduced easily from Figure 9, which illustrates the reconstruction of the cruciate ligament of the knee with the method which uses the semitendinosus and gracilis tendons T, which are engaged on the tibial side to the respective anchor 1 and are fixed to the femur on the opposite side by means of staples 15.

As can be seen, the positioning of the anchor in the tibial bore is ensured by the straight portions 4, 5, which guide the insertion of the ring element 2 until the flange 3 abuts against the cortex.

The described invention perfectly achieves the intended aim and object. It is significantly important that the flange 3, thanks to the articulation of the portions 8 and 9 in the seats 13 and 14, once the tension of the tendons has been adjusted to the chosen value, rests perfectly on the tibial cortex, so as to avoid load stresses in highly localized regions which might otherwise cause failures of the bone and compromise the preset tensioning of the tendons.

Numerous modifications and variations are possible in the practical embodiment of the invention, all of which are within the scope of the appended claims. Thus, for example, the portions 8, 9 of the ring element 2 can be retained by interference in the seats 13, 14 of the flange 3, despite allowing the articulation of said flange with respect to the element 2.

It is further possible to insert the anchor 1 in the femoral bore on its own or in combination with the anchor inserted in the tibial bore.

The disclosures in Italian Utility Model Application No. BO2006U000008 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An anchor for tendons used in the reconstruction of a ligament, particularly of the cruciate ligament of the knee, **characterized in that** it comprises a ring element (2) which can be inserted in a bore formed in the tibia or in the femoral condyle of the knee and an annular flange (3) for abutment against the region that surrounds the opening of the bore in the condyle in which said ring element (2) has been inserted, said ring element (2) being provided, on opposite sides, with two portions (8, 9) which protrude from it and are mutually aligned so as to be external to the cortex when said ring element is inserted in said bore, said annular flange (3) being provided with an opening (11) whose breadth is such as to allow said ring element (2) to pass through it and allow said protrusions (8, 9) to engage on said flange (3) in diametrically opposite positions.

2. The anchor according to claim 1, **characterized in that** said ring element (2) forms an eye which has substantially the shape of an elongated ellipse composed of two straight and parallel portions (4, 5) which are mutually connected by two semicircular portions (6, 7).

3. The anchor according to claim 1 or 2, **characterized in that** said portions (8, 9) are shaped like cylindrical rods which are tangent to a semicircular portion (7) and perpendicular to the straight portions (4, 5) and have a circular cross-section whose diameter is equal to the thickness of the ring element.

4. The anchor according to one of claims 1 to 3, **characterized in that** said annular flange (3) is constituted by a washer (10) which encloses a central opening (11) whose diameter is slightly larger than the external distance between said parallel portions (4, 5) of the ring element (2), said washer (10) having a flat face for resting against the cortex of the tibia or of the femoral or tibial condyle, and a cambered upper face (12) in which there are two mutually diametrically aligned notches (13, 14), which are substantially semicylindrical and have such dimensions as to act as seats adapted to accommodate articulately said rod-like portions (8, 9) of the ring element (2).

5. The anchor according to one of the preceding claims, **characterized in that** said ring element (2) has a substantially elliptical cross-section.

6. The anchor according to one of the preceding claims, **characterized in that** said portions (8, 9) are retained by interference in the respective seats (13, 14) so as to allow the articulation of the ring element (2) with respect to said flange (3).
